# EUROPEAN PATENT SPECIFICATION

(11) **EP 1 803 418 B1**
(45) Date of publication and mention of the grant of the patent: **22.10.2008**
(21) Application number: 06256636.9
(22) Date of filing: 29.12.2006
(51) Int. Cl.: A61F 2/06, A61F 2/90

(54) **D-shape stent for treatment of abdominal aortic aneurysm**
D-förmiger Stent zur Behandlung von Abdominalaneurismen
Stent en forme de D pour anévrisme de l'aorte abdominale

(30) Priority: 30.12.2005 US 323013
(43) Date of publication of application: 04.07.2007
(73) Proprietor: Cordis Corporation, Miami Lakes, FL 33014 (US)
(72) Inventor: Park, Jin S., Parsippany, NJ 07054 (US)
(74) Representative: Gover, Richard Paul

(56) References cited:
- WO-A-20/04096092
- US-A- 6 070 589
- US-A- 6 129 756
- US-B1- 6 730 119

## Description

The invention relates to the field of medical devices, and more specifically to a prosthesis for the treatment of vascular disease, particularly abdominal aortic aneurysm.

Vascular disease is a leading cause of premature mortality in developed nations, often presenting as a vascular aneurysm. A vascular aneurysm is a localized dilation of a vessel wall, due to thinning or weakness of the wall structure, or separation between layers of the vessel wall. If untreated, the aneurysm may burst and hemorrhage uncontrollably. Aneurysms are particularly dangerous and prevalent in the aorta, because the aorta supplies blood to all other areas of the body, and because the aorta is subject to particularly high pressures and stresses accordingly. Rupture of an aortic aneurysm is the 15^{th} leading cause of death the United States, afflicting 5% of older men.

Aortic aneurysms are described by their position. They are either thoracic, generally between the aortic arch and the junction of the left and right renal arteries, or abdominal, between the junction of the renal arteries and the branch of the iliac arteries.

It is known to treat aortic aneurysms surgically where blood pressure control medication is unsuccessful at arresting growth of the aneurysm. Surgery often involves the insertion of a vascular stent graft to exclude the aneurysm and carry blood past the dilated portion of the vessel, relieving the pressure on the aneurysm. Designing a viable stent graft for the treatment of abdominal aortic aneurysm (AAA) is particularly challenging, in part because the graft must branch to follow the shape of the abdominal aorta to carry blood into the separate iliac arteries without obstruction. Moreover, it would be advantageous to design a stent graft that is collapsible to facilitate percutaneous insertion by minimally invasive surgical techniques.

US-6070589 discloses a method for deploying a bypass graft stent. The method comprises introducing first and second graft stent through respective first and second access sites on a first side of pathological defect, advancing the graft stents until each extends across the defect and is positioned within a common body passageway on a second side of the pathological defect, and drawing the graft stents together within the common body passageway.

WO-2004/096092 discloses a vascular graft and a deployment system for treating an abdominal aneurysm at the bifurcation of the aorta and iliac arteries. A tubular implant having a proximal section, a distal section and a hinged connection therebetween is positioned across the birfurcation such that the proximal section extends to a first iliac and the distal section extends into the second iliac. The implant is folded at the hinge to form a bifurcated implant.

US-6730119 discloses an abdominal aortic aneurysm exclusion device with iliac vessel extensions. A sectional bifurcated stent is introduced in sections into the vasculature and assembled in situ to form a scaffold for the introduction of material to occlude the aneurysm cavity.

The scope of the present invention is defined by the appended claims.

Provided according to the present invention is a method of forming a prosthetic stent, and a stent formed according to the method. The method includes providing a stent having a semicircular portion and a diameter portion connecting the ends of the semicircular portion. The stent may be cut, for example, laser cut, from a unitary cylinder of material, preferably a shape memory material and more preferably Nitinol or a Nitinol alloy, and may be shape-set in the shape of a semicircular portion and a diameter portion connecting the ends of the semicircular portion. A compressive force is applied to the diameter portion, which is then heated, preferably to between about 375 and about 650 degrees C, and more preferably between about 400 degrees C and about 600 degrees C, while under the compressive force. Heat may be applied by one or more of resistance heating, air heating, laser heating, induction heating, and hot die application. Upon releasing the compressive force, the ends of the semicircular portion hold the diameter portion in tension.

Also provided according to the present invention is a prosthetic stent having a semicircular portion, and a diameter portion connecting the ends of the semicircular portion, the diameter portion being held in tension by the ends of the semicircular portion. The prosthetic stent may include a vascular graft surrounding the stent. The prosthetic stent preferably comprises a shape memory material, more preferably Nitinol or a Nitinol alloy. The prosthetic stent may be comprised of a plurality of struts arranged in a repeating diamond pattern.

Also provided according to the present invention is a bifurcated prosthetic stent graft for a bifurcated lumen, the bifurcated prosthetic stent graft having two stent grafts with co-located respective first ends, and disparately located respective second ends. Each of the two stent grafts includes a first stent segment at the first end of the stent graft having a semicircular portion and a diameter portion connecting the ends of the semicircular portion, the diameter portion being held in tension by the ends of the semicircular portion such that the first stent segment defines a substantially D-shape. One or more transition segments transitions the stent graft between a substantially D-shape on one end and a substantially circular shape on the opposite end. The opposite end of the transition segment is in communication with the second end of the stent graft. A vascular graft encloses the first stent segment and one or more transition stent segments, the vascular graft providing a fluid flow lumen from a first end of the stent graft to the second end of the stent graft.

Embodiments of the invention will now be described by way of example with reference to the accompanying drawings, wherein like reference numerals refer to like structures across the several views, and in which:
Figs. 1A, 1B, 1C and 1D illustrate a single D-shape stent according to an embodiment of the present invention in perspective view, side elevation view, plan view, and front elevation view, respectively;
Figs. 2A, 2B, 2C and 2D illustrate a double D-shape stent assembly according to an embodiment of the present invention in perspective view, side elevation view, plan view, and front elevation view, respectively;
Fig. 3 illustrates a stent graft for the treatment of abdominal aortic aneurysm according to the present invention;
Figs. 4A and 4B illustrate the effect of radial compression on a D-shape stent according to less preferred embodiments of a D-shape stent; and
Fig. 5 illustrates the effect of radial compression on a D-shape stent according to a preferred embodiment of the present invention.

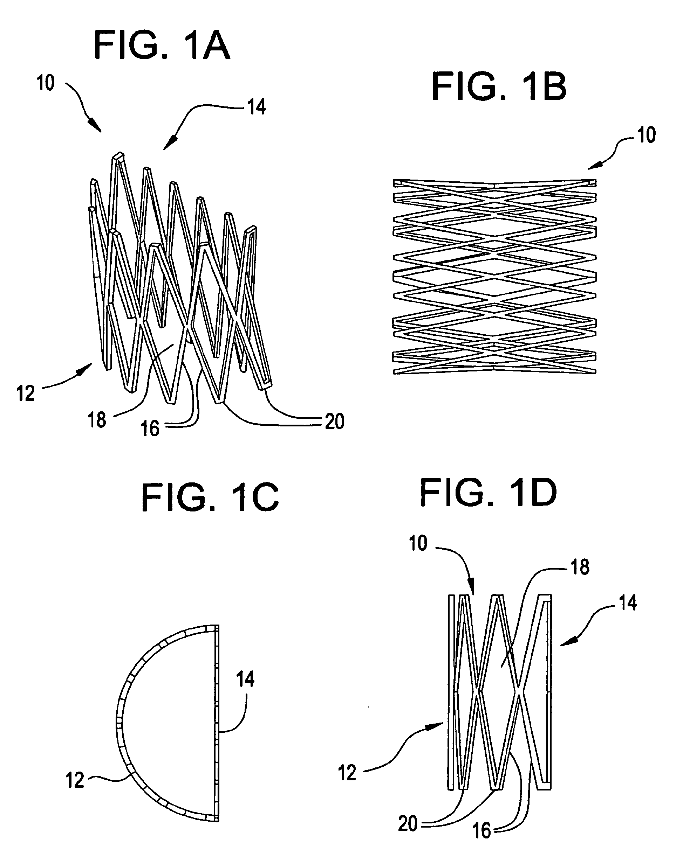

Referring now to Figs. 1A-1D, illustrated is a single D-shape stent, generally 10, according to the present invention. The stent 10 has a semicircular portion 12, and a diameter portion 14 connecting the ends of the semicircular portion 12. The stent 10 is formed of plural struts 16. The struts 16 are arranged in a diamond pattern, where four adjacent struts 16 define the borders of a diamond 18, with this pattern repeating in the stent 10. However, other strut patterns are known in the art and may be substituted without departing from the present invention.

D-shape stent 10 preferably comprises a shape memory material, a group that includes, but is not limited to, Nitinol or a Nitinol alloy, examples of the latter including Nitinol Niobium (NiTi-Nb), Nitinol Platinum (NiTi-Pt), or Nitinol Tantalum (NiTi-Ta). D-shape stent 10 can be formed by cutting the stent from a cylindrical tube of Nitinol or a Nitinol alloy, for example by a laser-cutting technique as is known in the art, and shape-setting the stent 10 into D-shape as shown.

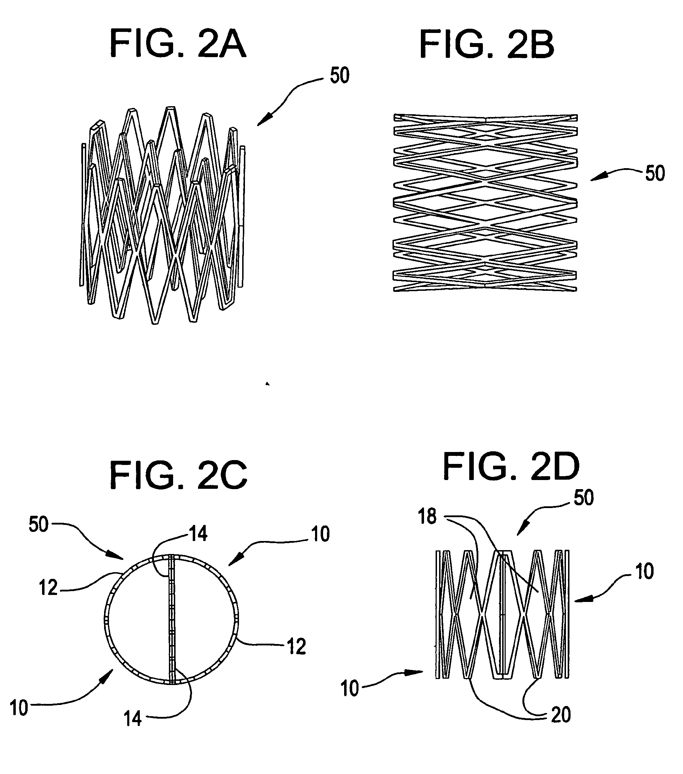

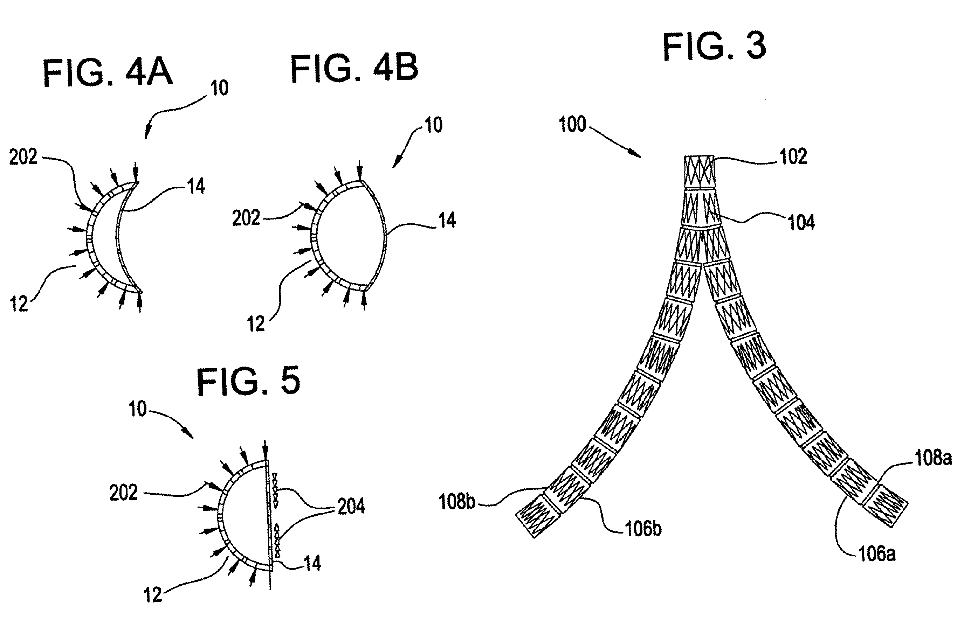

Referring now to Figs. 2A-2D, illustrated is a double D-stent assembly, generally 50, according to the present invention. The double D-stent assembly 50 is comprised of two individual D-stents 10, oriented with their respective diameter portions 14 adjacent and touching. One or more barbs at or near the respective diameter portions 14 of each D-Shape stent 10 and extending outward from the diameter portion 14 may be provided to interlock with the stent structure or associated graft of the other and resist migration. The two D-stents 10 can be located in the abdominal aorta of a patient to form the structure of a circular inlet to a bifurcated AAA stent graft 100 (See Fig. 3). Accordingly, the two branches 106a, 106b, of the AAA stent graft 100 can be compressed to a low profile, and introduced to the aorta percutaneously, with all the associated advantages of minimally invasive surgical methods and techniques.

The diamonds 18 of each D-shape stent 10 have points 20 at the bottom where additional stent segments can be attached or at least generally aligned. The additional segments will either transition from the substantially D-shape of the stent 10 to a circular shape, or be circular shaped.

Referring now to Fig. 3, a bifurcated AAA stent graft, generally 100, is illustrated. Two D-stents 10 positioned as in assembly 50 form the first segment 102 of the stent graft 100. Thereafter, transition segment 104 transitions between the substantially D-shape of stents 10 and the circular shape of branch 106a, 106b. Each branch 106a, 106b, includes a vascular graft 108a, 108b, carried by and surrounding D-shape stents 10, and additional transition or circular stent segments, to provide a fluid flow path through the respective branch 106a, 106b.

Referring now to Figs. 4A and 4B, when inserted in the aorta, the semicircular portion 12 will be subjected to radially inward compressive stress by the vessel wall, represented by arrows 202. Absent measures such as those according to the present invention, this compression could induce a bowing or buckling of the diameter portion 14, as the compressive force reduces the distance between the ends of the semicircular portion 12, as illustrated in Figs. 4A, 4B.

According to the present invention, the diameter portion 14 is placed under tension in the deployed shape. This is accomplished, for example, by applying a compression force to the diameter portion 14 during manufacture of the D-shape stent 10. While under compression, a localized heat treatment is applied only to only the diameter portion 14, while avoiding any heating of the semicircular portion 12. Contemplated methods of heating include, but are not limited to, hot die application, resistance heating, induction heating, laser heating, or application of heated air to the diameter portion 14. Where D-shape stent 10 is made of a Nitinol or a Nitinol alloy material, a preferred range of heating is between about 375-650 degrees C, and more preferably between about 400-600 degrees C. The heating alters the molecules of the diameter portion 14 to relive the compressive stress resulting from the applied compression force.

Moreover, the heat application is preferably localized or isolated to only the diameter portion 14. Towards this end, a heat sink can be used adjacent or near the semicircular portion 12 to direct heat away from or draw heat from the semicircular portion 12.

Following the heat treatment, having removed the influence of the compressive force, the ends of semicircular portion 12 of D-shape stent 10 hold the diameter portion 14 under tension. Referring now to Fig. 5, arrows 204 illustrate the tension in diameter portion 14 of stent 10. When the semicircular portion 12 is subjected to radial compressive force, illustrated by arrows 202, the tension in diameter portion 14 is relieved, but no buckling or bowing occurs. Accordingly, a D-shape stent 10 according to the present invention has greater dimensional stability in use.

## Claims

1. A prosthetic stent (10) comprising:
a semicircular portion (12);
a diameter portion (14) connecting the ends of the semicircular portion (12),
**characterised by** the diameter portion (14) being held in tension by the ends of the semicircular portion (12).

2. The prosthetic stent (10) according to claim 1, further comprising a vascular graft surrounding the stent (10).

3. The prosthetic stent (10) according to claim 1, further comprising one or more barbs at or near the diameter portion (14) and extending outward from the diameter portion (14).

4. The prosthetic stent (10) according to claim 1, wherein the stent (10) further comprises a vascular graft carried by the stent (10).

5. The prosthetic stent (10) according to claim 1, wherein the stent (10) comprises a shape memory material.

6. The prosthetic stent (10) according to claim 5, wherein the shape-memory material comprises Nitinol or a Nitinol alloy.

7. The prosthetic stent (10) according to claim 1, further comprising a plurality of struts (16) arranged in a repeating diamond pattern.

8. A bifurcated prosthetic stent graft (100) for a bifurcated lumen, the bifurcated prosthetic stent graft (100) comprising two stent grafts having co-located respective first ends, and disparately located respective second ends, each of the two stent grafts comprising:
a first stent segment (10) at the first end of the stent graft, the first stent segment comprising a prosthetic stent (10) according to claim 1 such that the first stent segment (10) defines a substantially D-shape;
one or more transition segments (104) which transition between a substantially D-shape on one end and a substantially circular shape on the opposite end, the opposite end of the transition segment (104) being in communication with the second end of the stent graft; and
a vascular graft (108a, 108b) enclosing the first stent segment (10) and one or more transition stent (104) segments, the vascular graft (108a, 108b) providing a fluid flow lumen from a first end of the stent graft to the second end of the stent graft.

9. A method of forming a prosthetic stent (10) comprising:
(a) providing a stent (10) having a semicircular portion (12) and a diameter portion (14) connecting the ends of the semicircular portion (12);
**characterised by**
(b) applying a compressive force to the diameter portion (14);
(c) applying heat to the diameter portion (14) while under the compressive force; and
(d) releasing the compressive force such that the ends of the semicircular portion (12) hold the diameter portion in tension (14).

10. The method according to claim 9, wherein the providing step further comprises shape-setting the stent (10) in the shape of a semicircular portion (12) and a diameter portion (14).

11. The method according to claim 9, wherein the providing step further comprises cutting the stent (10) from a unitary cylinder of material.

12. The method according to claim 9, wherein the providing step further comprises providing the stent (10) comprising a shape memory material.

13. The method according to claim 12, wherein the providing step further comprises providing the stent (10) comprising Nitinol or a Nitinol alloy.

14. The method according to claim 9, wherein the step of applying heat further comprises applying heat to a temperature of between about 375°C and about 650°C,

15. The method according to claim 14, wherein the step of applying heat further comprises applying heat to a temperature of between about 400 °C and about 600°C.

16. The method according to claim 9, wherein the step of applying heat further comprises applying heat by one or more of resistance heating, induction heating, laser heating, air heating, and hot die application.

17. The method according to claim 9, wherein the step of applying heat further comprises isolating the application of heat to only the diameter portion (14) and not to the semicircular portion (12).

18. The method according to claim 17, wherein the step of applying heat further comprises providing a heat sink to direct heat away from or draw heat from the semicircular portion (12).

## Patentansprüche

1. Prosthetischer Stent (10), der Folgendes umfasst:
einen halbrunden Abschnitt (12);
einen Durchmesserabschnitt (14), der die Enden des halbrunden Abschnitts (12) verbindet, **dadurch gekennzeichnet, dass** der Durchmesserabschnitt (14) von den Enden des halbrunden Abschnitts (12) unter Spannung gehalten wird.

2. Prosthetischer Stent (10) nach Anspruch 1, der weiterhin ein vaskuläres Transplantat umfasst, das den Stent (10) umgibt.

3. Prosthetischer Stent (10) nach Anspruch 1, der weiterhin einen oder mehrere Widerhaken an oder nahe dem Durchmesserabschnitt (14) aufweist und die sich von dem Durchmesserabschnitt (14) nach außen erstrecken.

4. Prosthetischer Stent (10) nach Anspruch 1, wobei der Stent (10) weiterhin ein vaskuläres Transplantat aufweist, das von dem Stent (10) getragen wird.

5. Prosthetischer Stent (10) nach Anspruch 1, wobei der Stent (10) weiterhin ein Material mit Formgedächtnis umfasst.

6. Prosthetischer Stent (10) nach Anspruch 5, bei welchem das Material mit Formgedächtnis Nitriol oder eine Nitriol-Legierung umfasst.

7. Prosthetischer Stent (10) nach Anspruch 1, der weiterhin mehrere Streben (16) umfasst, die in einem sich wiederholenden Rautenmuster angeordnet sind.

8. Gabelförmiges prosthetisches Stenttransplantat (100) für ein sich gabelndes Lumen, wobei das gabelförmig prosthetische Stenttransplantat (100) zwei Stenttransplantate umfasst, die gemeinsam angeordnete jeweilige erste Enden und getrennt angeordnete jeweilige zweite Enden aufweist, wobei jedes der zwei Stenttransplantate Folgendes umfasst:
ein erstes Stentsegment (10) an dem ersten Ende des Stenttransplantates, wobei das erste Segment einen prosthetischen Stent (10) nach Anspruch 1 umfasst, derart, dass das erste Stentsegment (10) im Wesentlichen eine D-Form definiert;
ein oder mehrere Übergangssegmente (104), welche zwischen einer im Wesentlichen D-Form an einem Ende und einer im Wesentlichen runden Form an dem gegenüberliegenden Ende übergehen, wobei das gegenüberliegende Ende des Übergangssegments (104) in Verbindung mit dem zweiten Ende des Stenttransplantats steht; und
ein vaskuläres Transplantat (108a, 108b), welches das erste Stentsegment (10) und ein oder mehrere Übergangsstentsegmente (104) einschließt, wobei das vaskuläre Transplantat (108a, 108b) ein Fluidstromlumen von einem ersten Ende des Stenttransplantates zu dem zweiten Ende des Stenttransplantates bereitstellt.

9. Verfahren zum Bilden eines prosthetischen Stents (10), das Folgendes umfasst:
(a) Bereitstellen eines Stents (10) mit einem halbrunden Abschnitt (12) und einem Durchmesserabschnitt (14), welcher die Enden des halbrunden Abschnitts (12) verbindet;
**gekennzeichnet durch**
(b) Anlegen einer Kompressionskraft an den Durchmesserabschnitt (14);
(c) Anwenden von Wärme auf den Durchmesserabschnitt (14) während dieser unter der Kompressionskraft steht; und
(d) Lösen der Kompressionskraft derart, dass die Enden des halbrunden Abschnitts (12) den Umfangsabschnitt (14) unter Spannung halten.

10. Verfahren nach Anspruch 9, bei welchem der Schritt zum Bereitstellen weiterhin ein Justieren der Form des Stents (10) in die Form eines halbrunden Abschnitts (12) und eines Durchmesserabschnitts (14) umfasst.

11. Verfahren nach Anspruch 9, bei welchem der Schritt zum Bereitstellen weiterhin ein Schneiden des Stents (10) aus einem einheitlichen Zylinder eines Materials umfasst.

12. Verfahren nach Anspruch 9, bei welchem der Schritt zum Bereitstellen weiterhin das Bereitstellen eines Stents (10) mit einem Material mit Formgedächtnis umfasst.

13. Verfahren nach Anspruch 12, bei welchem der Schritt zum Bereitstellen weiterhin das Bereitstellen des Stents (10) mit Nitriol oder einer Nitriol-Legierung umfasst.

14. Verfahren nach Anspruch 9, bei welchem der Schritt zum Anwenden der Wärme weiterhin ein Anwenden von Wärme bei einer Temperatur zwischen etwa 375°C und etwa 650°C umfasst.

15. Verfahren nach Anspruch 14, bei welchem der Schritt zum Anwenden der Wärme weiterhin ein Anwenden der Wärme bei einer Temperatur zwischen etwa 400°C und etwa 600°C umfasst.

16. Verfahren nach Anspruch 9, bei welchem der Schritt zum Anwenden der Wärme weiterhin ein Anwenden von Wärme durch Widerstandsheizen, Induktionsheizen, Laserheizen, Luftheizen und/oder durch eine Heißformanwendung umfasst,

17. Verfahren nach Anspruch 9, bei welchem der Schritt zum Anwenden der Wärme weiterhin das Isolieren der Anwendung von Wärme auf nur den Durchmesserabschnitt (14) und nicht auf den halbrunden Abschnitt (12) umfasst.

18. Verfahren nach Anspruch 17, bei welchem der Schritt zum Anwenden der Wärme weiterhin das Bereitstellen eines Kühlkörpers umfasst, um Wärme von dem halbrunden Abschnitt (12) wegzuleiten oder diesem zu entziehen.

## Revendications

1. Endoprothèse vasculaire (10) comprenant :
une partie semi-circulaire (12) ;
une partie diamétrale (14) raccordant les extrémités de la partie circulaire (12), **caractérisée en ce que** la partie diamétrale (14) est maintenue sous tension par les extrémités de la partie semi-circulaire (12).

2. Endoprothèse vasculaire (10) selon la revendication 1, comprenant en outre un greffon vasculaire entourant l'endoprothèse vasculaire (10).

3. Endoprothèse vasculaire (10) selon la revendication 1, comprenant en outre un ou plusieurs ardillons au niveau ou près de la partie diamétrale (14) et s'étendant vers l'extérieur depuis la partie diamétrale (14).

4. Endoprothèse vasculaire (10) selon la revendication 1, dans laquelle l'endoprothèse vasculaire (10) comprend en outre un greffon vasculaire porté par l'endoprothèse vasculaire (10).

5. Endoprothèse vasculaire (10) selon la revendication 1, dans laquelle l'endoprothèse vasculaire (10) comprend un matériau à mémoire de forme.

6. Endoprothèse vasculaire (10) selon la revendication 5, dans laquelle le matériau à mémoire de forme comprend du nitinol ou un alliage de nitinol.

7. Endoprothèse vasculaire (10) selon la revendication 1, comprenant en outre une pluralité d'entretoises (16) agencées suivant un motif de diamant répétitif.

8. Greffon d'endoprothèse vasculaire bifurqué (100) pour une lumière bifurquée, le greffon d'endoprothèse vasculaire bifurqué (100) comprenant deux greffons d'endoprothèse vasculaire présentant des premières extrémités respectives copositionnées, et des secondes extrémités respectives situées de façon disparate, chacun des deux greffons d'endoprothèse vasculaire comprenant :
un premier segment d'endoprothèse vasculaire (10) à la première extrémité du greffon d'endoprothèse vasculaire, le premier segment d'endoprothèse vasculaire comprenant une endoprothèse vasculaire (10) selon la revendication 1 de telle sorte que le premier segment d'endoprothèse vasculaire (10) définit un forme sensiblement en D ;
un ou plusieurs segments de transition (104) qui effectuent une transition entre une forme sensiblement en D sur une extrémité et une forme sensiblement circulaire sur l'extrémité opposée, l'extrémité opposée du segment de transition (104) étant en communication avec la seconde extrémité du greffon d'endoprothèse vasculaire ; et
un greffon vasculaire (108a, 108b) enfermant le premier segment d'endoprothèse vasculaire (10) et un ou plusieurs segments d'endoprothèse vasculaire de transition (104), le greffon vasculaire (108a, 108b) fournissant une lumière d'écoulement de fluide d'une première extrémité du greffon d'endoprothèse vasculaire à la seconde extrémité du greffon d'endoprothèse vasculaire.

9. Procédé de formation d'une endoprothèse vasculaire (10) comprenant les étapes consistant à :
(a) fournir une endoprothèse vasculaire (10) présentant une partie semi-circulaire (12) et une partie diamétrale (14) raccordant les extrémités de la partie semi-circulaire (12) ; **caractérisé par** les étapes consistant à :
(b) appliquer une force de compression sur la partie diamétrale (14) ;
(c) appliquer de la chaleur sur la partie de diamètre (14) alors qu'elle subit une force de compression ; et
(d) libérer la force de compression de telle sorte que les extrémités de la partie semi-circulaire (12) maintiennent la partie diamétrale sous tension (14).

10. Procédé selon la revendication 9, dans lequel l'étape de fourniture comprend le réglage de la forme de l'endoprothèse vasculaire (10) suivant la forme d'une partie semi-circulaire (12) et d'une partie diamétrale (14).

11. Procédé selon la revendication 9, dans lequel l'étape de fourniture comprend l'étape de découpage de l'endoprothèse vasculaire (10) à partir d'un cylindre unitaire de matériau.

12. Procédé selon la revendication 9, dans lequel le fourniture comprend en outre la fourniture de l'endoprothèse vasculaire (10) comprenant un matériau à mémoire de forme.

13. Procédé selon la revendication 12, dans lequel l'étape de fourniture comprend la fourniture de l'endoprothèse vasculaire (10) comprenant du nitinol ou un alliage de nitinol.

14. Procédé selon la revendication 9 dans lequel l'étape consistant à appliquer de la chaleur comprend en outre l'application de chaleur à une température comprise entre environ 375°C et environ 650°C.

15. Procédé selon la revendication 14, dans lequel l'étape consistant à appliquer de la chaleur comprend en outre l'application de chaleur à une température comprise entre environ 400 °C et environ 600 °C.

16. Procédé selon la revendication 9 dans lequel l'étape consistant à appliquer de la chaleur comprend en outre l'application de chaleur par une ou plusieurs parmi les applications de chauffage par résistance, chauffage par induction, chauffage au laser, chauffage d'air, et thermosoudage.

17. Procédé selon la revendication 9 dans lequel l'étape consistant à appliquer de la chaleur comprend en outre l'isolation de l'application de chaleur sur uniquement la partie diamétrale (14) et non la partie semi-circulaire (12).

18. Procédé selon la revendication 17, dans lequel l'étape consistant à appliquer de la chaleur comprend en outre la fourniture d'un dissipateur thermique pour évacuer la chaleur ou attirer la chaleur de la partie semi-circulaire (12).
